Europäisches Patentamt

**European Patent Office**

**Office européen des brevets**

(11) Publication number: **0 230 574**
**A2**

## (12) EUROPEAN PATENT APPLICATION

(21) Application number: **86116850.8**

(22) Date of filing: **04.12.86**

(51) Int. Cl.⁴: **A61K 39/395** , A61K 31/70 ,
A61K 31/715 , A61K 37/02 ,
//(A61K37/02,31:56)

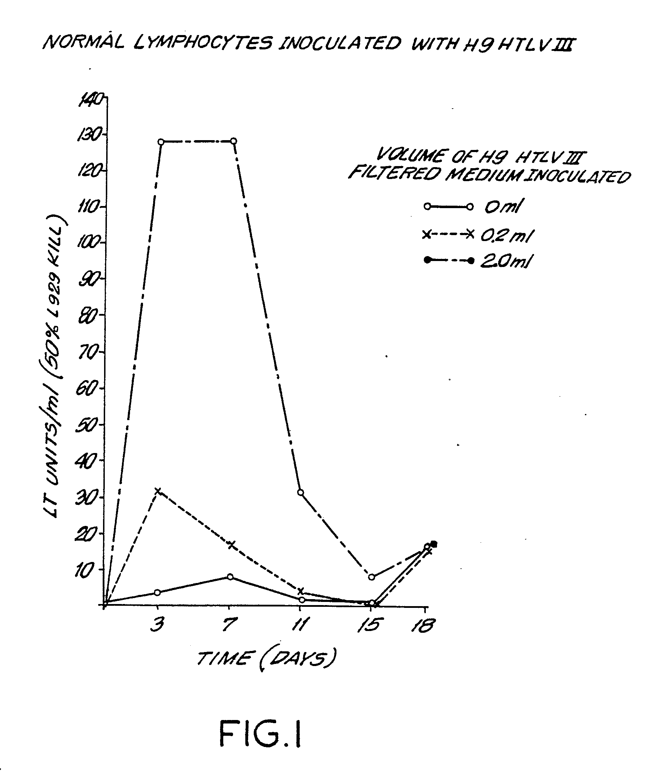

A request for correction of the drawings (Fig. 1, 2 and 3) has been filed pursuant to Rule 88 EPC. A decision on the request will be taken during the proceedings before the Examining Division (Guidelines for Examination in the EPO, A-V, 2.2).

(30) Priority: **31.01.86 US 825013**

(43) Date of publication of application:
**05.08.87 Bulletin 87/32**

(84) Designated Contracting States:
**AT BE CH DE ES FR GB GR IT LI NL SE**

(71) Applicant: **Yale University**
**60 College Street**
**New Haven Connecticut 06510(US)**

(72) Inventor: **Ruddle, Nancy H., Dr.**
**341 Willow Street**
**New Haven Connecticut 06511(US)**

(74) Representative: **Hansen, Bernd, Dr.rer.nat. et al**
**Hoffmann, Eitle & Partner Patentanwälte**
**Arabellastrasse 4**
**D-8000 München 81(DE)**

(54) **Pharmaceutical compositions against infections caused by LAV/HTLV III virus and the use thereof.**

(57) Compositions for treating a patient infected with LAV/HTLV III virus, said compositions inhibiting the production and/or inhibiting the activity of mononuclear cell derived cytotoxic factors, without permanently interfering with other immune cell functions.

EP 0 230 574 A2

## PHARMACEUTICAL COMPOSITIONS AGAINST INFECTIONS CAUSED BY LAV/HTLV III VIRUS AND THE USE THEREOF

### BACKGROUD OF THE INVENTION

The present invention concerns compositions for treating patients infected with LAV/HTLV III virus as well as the use of respective compositions for the preparation of a pharmaceutical. More particularly, the present invention relates to inhibiting the production and/or inhibiting the activity of mononuclear cell derived cytotoxic factors, e.g. lymphotoxin ("LT") and tumor necrosis factor ("TNF").

The etiological agent of acquired immunodeficiency syndrome ("AIDS") is a retrovirus called lymphadenopathy-associated virus (LAV) (F. Barre-Sinoussi, J. C. Chermann, F. Rey, M. T. Nugeyre, S. Chamaret, J. Gruest, C. Dauguet, C. Axler-Blin, F. Vezinet-Brun, C. Rouzioux, W. Rozenbaum and L. Montagnier, Science, 220, 868-870 (1983)) or human T lymphotropic virus III (HTLV III) (M. Popovic, M. G. Sarngadharan, E. Read and R. C. Gallo, Science, 224, 497-508 (1984)). The best evidence supports the concept that these are either the same virus or closely related variants (L. Ratner, W. Haseltine, R. Patarca et al, Nature , 313, 227-285 (1985)). The virus infects OKT4$^+$ cells, the subset predominately concerned with helper/inducer functions (D. Klatzmann, F. Barre-Sinoussi, M. T. Nugeyre, C. Dauguet, E. Vilmer, C. Griscelli, F. Vezinet-Brun, C.Rouzioux, J. -C. Gluckman, J. C. Chermann and L. Montagnier, Science, 225, 59-63 (1984)). In fact, the receptor for the virus appears to be the OKT4 molecule itself (D. Klatzmann, E. Champagne, S. Chamaret, J. Gruest, D. Guetard, T. Hercend, J. -C. Gluckman and L. Montagnier, Nature - (London), 312, 767-768 (1984); A. G. Dalgleish, P. C. L. Beverly, P. R. Clapham, D. H. Crawford, M. F. Greaves and R. A. Weiss, Nature (London), 312, 763-767 (1984)). As expected, therefore, the OKT8$^+$ subset of T cells, which expresses cytotoxic/suppressor functions, is not infected by LAV/HTLV III, nor is it seriously affected by it. =

Other investigations in this field are as follows:

L. Montagnier, C. Dauguet, C. Axler, S. Chamaret, J. Gruest, M. T. Nugeyre, F. Rey, F. Barre-Sinoussi and J. C. Chermann, Ann. Virol., 135E., 119 (1984); E. Vilmer, C. Rouzioux, F. Vezinet-Brun, A. Fischer, J. C. Chermann, F. Barre-Sinoussi, C. Gazengel, C. Dauguet, P. Manigne, C. Griscelli and L. Montagnier, Lancet, 1, 753 (1984); J. A. Levy, A. D. Hoffman, S. M. Kraker, J. A. Landis, J. M. Shimabukuro and L. S. Oshiro, Science, 225, 840 (1984)).

Several reports have indicated that when OKT4$^+$ cells- are infected by LAV/HTLV-III in vitro, their function is severely inhibited (D. Klatzmann et al, Nature (London), (1984), supra). These cells die, due not to a "premature senescence" or inhibition of cell division, but as the result of a cytopathic effect (S. Harada, Y. Koyanagi and Y. Naoki, Science, 229, 563-566 (1985)). Clinical studies indicate that in AIDS a profound lymphopenia in the helper T-cell subset results in diminished immune capability (M. Seligmann, L. Chess, J. L. Fahey et al, N. Eng. J. Med., 311, 1286-1292 (1982)). One of the most puzzling aspects of the disease has been the mechanism of T-cell depletion. The genome of the AIDS virus has been sequenced and almost completely mapped, but there is yet no candidate virus-encoded molecule which could kill T cells. However, a virus-encoded molecule has been described which is capable of acting in a trans fashion and activating genes associated with long terminal repeat sequence (S.K. Arya, C. Guo, S. F. Josephs et al, Science, 229, 69-73 (1985)). These results suggest that genes coded in infected cells themselves could also be activated. There is considerable evidence that this can occur in the case of the related virus HTLV-I, the etiological agent of adult T cell leukemia. This retrovirus also encodes a trans acting protein (J. G. Sodroski, C.A. Rosen and W. A. Haseltine, Science, 225, 381-385 (1984)). Infection of T cells with HTLV-I results in an impressive increase in the number of receptors for interleukin 2 expressed by the cells (J. M. Depper, W. J. Leonard, M. Kronke et al, J. Immunol., 133, 1691-1695 (1984)). This may account in part for the absence of growth regulation of HTLV-I infected T cells and the development of the transformed state.

Lymphotoxin is produced by lymphocytes after stimulation with mitogen or specific antigen (N. H. Ruddle and B. H. Waksman, J. Exp. Med., 128, 1267-1269 (1968); G. A. Granger, and W. P. Kolb, J. Immunol., 101, 111-120 (1968); N. H. Ruddle, Immunol Today, 6, 156, 159 (1985)). Its production is thus a marker for T-cell activation. In the mouse both class II restricted cells (Lyt 1$^+$) (D.D. Eardley, F. W. Shen, R. K. Gershon et al, J. Immunol., 124, 1199-1202 (1980); B. S. Conta, M. B. Powell and N. H. Ruddle, J. Immunol., 130, 2231-2235 (1983)) analogous to OKT4 cells in man, and class I MHC restricted T cells (Lyt 2$^+$) (B. S. Conta, M. B. Powell and N. H. Ruddle, J. Immunol., 134, 2185-2190 (1985)) analogous to OKT8$^+$

cells in man, can make lymphotoxin if specific antigen is presented by MHC matched cells. The cellular origin of lymphotoxin in man has been studied less extensively, but it can be produced by both OKT4+ and OKT8+ cells after stimulation with phytohemagglutinin (E. Leopardi and W. Rosenau, Cell Immunol., 83, 73-82 (1984)).

A gene for human lymphotoxin has been cloned and expressed in E. coli (P. W. Gray, B.B. Aggarwal, C. N. Benton et al, Nature (London), 312, 721-724 (1984)). The protein has an apparent molecular weight of 17,000 d. The fact that lymphotoxin can form multimers accounts in part for the several different sized species previously described. Lymphotoxin kills malignantly transformed cells more effectively that normal cells (M. S. Meltzer and G. L. Bartlett, J. Natl. Cancer Inst., 49, 1439-1443 (1972)), but it also kills virus-infected cells (P. Eifel, A. Billingsley and Z. J. Lucas, Cell Immunol., 49, 1439-1443 (1979)) and embryo fibroblasts (Ruddle and Waksman, Exp. Med., (1968), supra). It is cytostatic or cytotoxic, depending on the concentration and the nature of the target cell. It kills T cells maintained in interleukin 2 very effectively. (M. B. Powell, B. S. Conta and N. H. Ruddle, Lymphokine Res., 4, 13-26 (1985)). It is striking that when cloned T cells are stimulated to make large quantities of lymphotoxin, they themselves are killed, (Conta, Powell and Ruddle, J. Immunol., (1985) supra ). It is not yet clear whether this is due to autodestruction or to their annihilation by large quantities of lymphotoxin produced by neighboring T cells.

There has been controversy in the scientific community regarding the nomenclature and definition of the terms tumor necrosis factor ("TNF") and lymphotoxin ("LT"). TNF and LT can generally be described as molecules produced by cells of the immune system that are cytotoxic to cells and cause necrosis of tumors in mice.

Tumor necrosis factor (TNF) was observed by E. A. Carswell et al, Proc. Natl. Acad. Sci. USA, 72, 3666-3670 (1975) during a study of the antitumor activity of sera from mice infected with bacillus Calmette-Guerin (BCG) and subsequently injected with endotoxin. These sera have been observed to cause the hemorrhagic necrosis and regression of certain mouse tumors in vivo . These sera were also found to have cytotoxic/cytostatic effects on mouse and human tumor cells in vitro (E. A. Carswell et al, supra; L. Helson et al, Nature (London), 258, 731-732 (1975); D. N. Mannel et al, Infect. Immun., 28, 204-211 (1980); F. C. Kull and P. Cuatrecasas, J. Immunol., 126, 1279-1283 (1981); K. Haranaka and N. Satomi, Jpn. J. Exp. Med., 51, 191-194 (1981)). A similar factor was found to be induced in rats (Carswell et al, supra ) and rabbits (Carswell et al, supra, N. Matthews and J. F. Watkins, Br. J. Cancer, 38, 302-309 (1978)); J. Ostrove and G. E. Gifford, Proc. Soc. Exp. Biol. Medl., 160, 354-358 (1979)).

Biochemical characterization has shown that mouse serum TNF exists in a least two forms: a 150,000 $M_r$ form, (Kull and Cuatrecasas, supra and S. Green et al, Proc. Natl., Acad. Sci. USA, 73, 381-385 (1976)) and a 40,000-60,000 $M_r$ form, D. N. Mannel et al, supra , Kull and Cuatrecasas, supra, and Haranaka and Satomi, supra. TNF in rabbit serum has been reported to have a molecular weight of 39,000 (N. Matthews et al, Br. J. Cancer, 42 , 416-422 (1980)) and 67,000 (M. R. Ruff and G. E. Gifford, J. Immunol., 125, 1671-1677 1980)).

Studies have indicated that both in vivo and in vitro activities of mouse TNF appear to be a property of the same molecule. The cellular source of TNF in the mouse was initially assumed to be the macrophage, because the agents used to prime for TNF production cause massive hyperplasia of macrophages in liver and spleen, (Carswell et al, supra). From studies of macrophage-rich cell populations in vitro, N. Matthews, Br. J. Cancer, 38, 310-315 (1978) and D. N. Mannel et al, supra, a similar conclusion was reached with regard to the source of mouse and rabbit TNF. Direct evidence that macrophages are at least one cell type in the mouse capable of producing TNF comes from studies with cloned lines of mouse histiocytomas. These cells constitutively produce low levels of TNF that are greatly increased after exposure to endotoxin.

B. D. Williamson et al, Proc. Natl. Acad. Sci. USA, 80, 5397-5401 (1983), described the capacity of human cell lines of hematopoietic origin to produce a factor with TNF activity. Evidence demonstrating that the molecule is a human TNF included the following: 1) the anticellular responses of a panel of human cell lines to human TNF, e.g., TNF (LuKII), or mouse TNF are indistinguishable and can be potentiated in a synergistic fashion by interferon, 2) mouse L cells made resistant to mouse TNF are resistant to human TNF, e.g., TNF(LuKII), 3) mouse L cells made resistant to human TNF, e.g., TNF(LuKII) are resistant to mouse TNF, and 4) human TNF, e.g., TNF(LuKII) causes hemorrhagic necrosis of meth A sarcoma in the standard in vivo TNF assay.

Recently French researchers have sought to treat patients suffering from AIDS by using cyclosporin, a substance used for transplant patients to suppress organ rejection. Cyclosporin by itself, however, has the disadvantage of suppressing a patient's immune system.

A recent article, Science, 230, December 20, 1985, pages 1355-1358, discussed strategies for possible treatments for AIDS patients.

There has been limited progress in the treatment of AIDS patients with agents that stimulate T-cell proliferation, such as interleukin 2, or agents which inhibit viral replication.

Heretofore, there has not been a successful method to treat AIDS victims.

## SUMMARY OF THE INVENTION

It is an object of the invention to provide compositions to treat patients infectd with LAV/HTLV III virus, without interfering with other immune cell functions.

This and other objects are provided by the present invention wherein a method is provided for treating a patient infected with LAV/HTLV III virus comprising inhibiting the production and/or inhibiting the activity of mononuclear cell (such mononuclear cells can be thymus derived lymphocytes or monocytes) derived cytotoxic factors, e.g., lymphotoxin and tumor necrosis factor or leukoregulin and natural killer cytotoxic factor ("NKCF"), without permanently interfering with other immune cell functions.

## BRIEF DESCRIPTION OF THE DRAWINGS

Fig. 1 is a plot of LT units/ml versus time (days) depicting the results of inoculating normal lymphocytes with three different volumes of HTLV III filtered inoculated medium.

Fig. 2 is a plot of total cells versus days for three different volumes of HTLV III filtered inoculated medium.

Fig. 3 is a plot of reverse transcriptase ("RT") versus days for two different volumes of HTLV III filtered inoculated medium.

## DETAILED DESCRIPTION OF THE INVENTION

Without wishing to be bound by any particular theory of operability, it is believed that the $\underline{trans}$ acting protein of LAV/HTLV III kills cells by inducing them to make a cellular cytotoxic protein, e.g., lymphotoxin - (T-cell-encoded cytotoxic protein), which in abnormally high quantities result in the cells' self-destruction. Lymphotoxin may even become serum detectable. It may induce immunosuppression and susceptibility to opportunistic infections by organisms such as $\underline{Pneumocystis}$ $\underline{carinii}$. Defects in tumor immunity may result from depletion of OKT4+ cells.

Patients with AIDS are frequently afflicted with a wasting syndrome (cachexia). Cachexia in AIDS patients may be due to the presence of abnormally high levels of lymphotoxin. Often the pathogenesis of this syndrome cannot be attributed to any known cause. It has been suggested that a similar cachexia associated with parasitic infections and cancer is due to a substance in the serum, termed "cachectin", now known to be the same as tumor necrosis factor (B. Beutler, D. Greenwald, S. D. Holmes, et al, $\underline{Nature}$ - $\underline{(London)}$, $\underline{316}$ (1985). It is believed that lymphotoxin also expresses cachectin activity, as assayed by inhibition of the enzyme lipoprotein lipase. It is believed that the cachexia of AIDS is due to a cytotoxic cellular protein; and that wasting is reversable by agents which reverse that activity. Accordingly, the present invention may serve to treat cachexia.

The methods of the present invention are based on interfering with certain cytotoxic factors, e.g., lymphotoxin, which when produced in high levels are believed to induce AIDS and AIDS related diseases. Thus, the methods of treatment described herein are based on two approaches.

One approach is to treat AIDS patients with an agent which interferes with cytotoxin, e.g., lymphotoxin activity, such as by employing a monoclonal (or polyclonal) antibody or an agent which blocks the receptor for lymphotoxin. The other approach, difficult at this time, but probably more promising in the long run, would be to interfere with the induction of cytotoxins, e.g., lymphotoxin from AIDS-virus infected cells, e.g., inhibiting lymphotoxin gene transcription. Regulatory sequences on the lymphotoxin gene and viral signals which activate or suppress the activity of such sequences will soon be identified. Once understood, another approach to anti-lymphotoxin therapy can be designed and applied to AIDS.

The present invention involves the following methods for inhibiting the activity of cytotoxic factors:

(1) introducing in a patient an effective amount of antibodies, polyclonal or monoclonal, to the cytotoxic factors; and

4

(2) blocking the cellular receptors of the cytotoxic factors, e.g., by introducing into a patient an effective amount of antagonist to the receptors, e.g., antibody to the receptor (polyclonal or monoclonal), monosaccharides, oligosaccharides, polysaccharides, and derivatives of monosaccharides, oligosaccharides and polysaccharides. Particularly preferred saccharides are mannose and galactose. Alternatively, a nonfunctional cytotoxin molecule can be utilized, i.e., a molecule that binds to the receptor, but has no cytotoxic activity (a molecule which competes with lymphotoxin for its receptor, for example, but has no cytotoxic activity).

The present invention also involves inhibition of production of cytotoxic factors by introduction into the patient an effective amount of (a) interleukin 2 and (b) cyclosporin, e.g., cyclosporin A, steroids or mixtures thereof.

Preferably, the cyclosporin, steroids or mixture thereof is administered first to a patient and when the cytotoxin, for example, lymphotoxin, level in a patient decreases, interleukin 2 is then administered to reconstitute (i.e., repair) the T-cell population which was depleted by the cyclosporin or steroids.

Steroids for use in the present invention would include those agents heretofore used to prevent organ transplant rejection, for example, imuran and prednisone.

The present invention can be applied to the treatment for AIDS, particularly the lymphopenia associated with the disease. Future use could include treatment of the neurological symptoms associated with the disease if such symptoms are induced by a cellular protein.

The invention will now be described with reference to the following non-limiting examples:

## Example 1

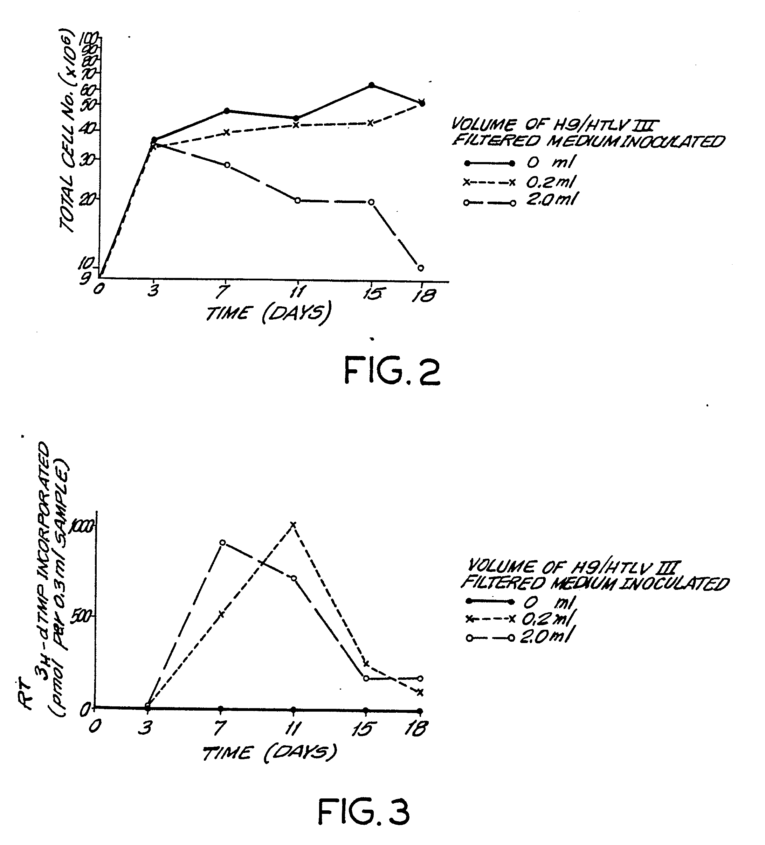

Lymphocytes were taken form a normal donor and infected with 0, 0.2 or 2.0 ml of supernatant fluid from an HTLV-III infected H9 (human T lymphocyte) culture (L. Ratner et al, supra). The supernatants of the lymphocyte cultures were collected at days 3, 7, 11, 15 and 18. The growth of the lymphocytes was evaluated (see Fig. 2), the virus content was assayed by reverse transcriptase using dA/dT or rA/dT as a template (see Fig. 3) and the lymphotoxin content of the supernatants was assayed (see Fig. 1). The latter was measured by killing L929 (mouse fibroblast line) (ATCC No. CCL 1) cells and was evaluated 72 hours after addition of culture supernatant. THe results of the experiment were as follows:

Lymphocytes uninfected with HTLV-III H9 supernatant increased in number over the course of the experiment and made no detectable virus and very little lymphotoxin.

Lymphocytes infected with 0.2 ml of HTLV-III H9 supernatant grew and replicated virus for a short time and for an unexplained reason, the cells were not killed. These cells made a low level of lymphotoxin.

Lymphocytes, infected with 2 ml of HTLV-III H9 initially grew and then died, beginning after day 3. They made virus which was detectable at day 7 and declined as they died. These cells made high titers of lymphotoxin which was apparent before cell death began and then declined on day 11, as the cells died.

The conclusion: the data are consistent with the hypothesis that HTLV-III turns on cellular lymphotoxin production and kills the cells.

## Example 2

Data obtained from an experiment provides evidence that production of lymphotoxin (LT) from activated T lymphocytes is inhibited by the drug cyclosporin A.

A murine T cell cloned line 153E11 is maintained in the presence of specific antigen (ovalbumin), interleukin 2 and syngeneic spleen cells. When this cell line is stimulated with antigen or a mitogenic plant lectin, such as concanavalin A, it secretes lymphotoxin into the culture supernatant. As reported in Table 1 hereinbelow, T cells were stimulated with concanavalin A in the presence and absence of two concentrations of cyclosporin A. Both concentrations inhibited LT production.

T cell clone 153E11 ($2 \times 10^6$ cells/ml) in 10 ml culture in RPMI medium (Gibco, Chagrin Falls, Ohio 44022, U.S.A.) supplemented with 10% fetal calf serum and 10 units IL2/ml was stimulated with concanavalin A (Sigma, St. Louis, Missouri, U.S.A.) at 5 $\mu$g/ml in the absence and in the presence of two concentrations of cyclosporin A (5 ng/ml or 5 $\mu$g/ml). Lymphotoxin production was measured 7 hours after initiation of the experiment by harvesting T cell culture supernatants. Serial dilutions of such supernatants were added to mouse L929 cells, the most susceptible targets of LT. One unit of LT is defined as that dilution which causes 50% cytotoxicity of L929 in the course of 72 hours.

5

The results summarized in Table 1 indicate that 153E11 T cell clone makes no LT in the absence of mitogen (concanavalin A) stimulation and makes 2048 μ/ml when stimulated. This titer is reduced when the cells are incubated with concanavalin A and cyclosporin A (5 ng/ml) to 416 U. This represents a 80% reduction. When stimulated cells are incubated in the presence of a higher concentration of cyclosporin A - (5 μg/ml) lymphotoxin production is completely inhibited, representing 100% reduction.

Concanavalin A induces lymphotoxin. Such lymphotoxin production is completely inhibitable by cyclosporin A.

## Table 1: Cyclosporin A Inhibits Production of Lymphotoxin by Activated T Cells

| Sample | Concanavalin A | Cyclosporin A | LT U/ml* | Reduction |
|--------|----------------|---------------|----------|-----------|
| 1 | -- | -- | 0 | -- |
| 2 | 5 μg | -- | 2048 | 0 |
| 3 | -- | 5 ng | -- | -- |
| 4 | -- | 5 μg | -- | -- |
| 5 | 5 μg | 5 ng | 416 | 80% |
| 6 | 5 μg | 5 μg | 0 | 100% |

* assayed by inhibition of L929 murine cell growth

   Application of 1 unit results in 50% cyctotoxicity

   $< 4$ U = 0

### Example 3

Individuals in the early stages of AIDS and individuals at risk for developing AIDS are possible subject for testing the methods of the present invention. Such individuals would be those having LAV/HTLV-III virus through documented exposure to the virus and/or are those demonstrating early symptoms of any of the syndromes associated with that virus. These include, but are not limited to individuals with AIDS related complex (ARC) individuals who are virus or virus antibody positive exhibiting neurological symptoms, and infants born of mothers with AIDS. The individuals will be treated with substances described in the application whose function would be to inhibit cytotoxin by (1) blocking cytotoxin activity or (2) blocking interaction of cytotoxin with its cellular receptor.

### (1) Inhibiting Cytotoxin By Blocking Cytotoxin Activity

Cytotoxin activity can be blocked by giving purified antilymphotoxin antibody as 6 hours perfusions in 250 ml normal saline for adults; 50 ml normal saline for infants. The dose of antibody will be within the range of 5 to 150 mg for adults given three times per week initially for 3 weeks and then once per week subsequently. The dose for infants will be no greater than 1.5 mg/kg.

## (2) Blocking Interaction Of Cytotoxin With Its Cellular Receptor

Cytotoxin interaction with its receptor will be inhibited with antagonists to the receptor.

(a) Purified monoclonal antibody to lymphotoxin receptor will be administered as 6 hour perfusions in normal saline. The dose will range from 5 to 150 mg in 250 ml and will be administered to adults three times weekly for three weeks and then once per week as maintenance therapy. The dose for infants will be no more than 1.5 mg/kg in 50 ml normal saline.

(b) Cytotoxin activity will be blocked by infusion of a substance which is a component of the receptor and will compete with the receptor for toxin binding. Individuals will be treated with mannose and/or galactosyl-containing substances by intravenous infusion three times per week for three weeks and once a week thereafter.

(c) Cytotoxin activity will be inhibited by competition with a "defective" lymphotoxin molecule. Such defective molecule is a molecule which contains only a portion of the cytotoxin, e.g., lymphotoxin molecule, e.g., lacking the N-terminal end, but which contains approximately 15 to 20 C-terminal amino acids. This "defective" molecule could be a mutant or a molecule prepared by cleaving, for example, by use of a protolytic enzyme, a full length cytotoxin molecule.·

It will be appreciated that the instant specification and claims are set forth by way of illustration and not limitation, and that various modifications and changes may be made without departure from the spirit and scope of the present invention.

## GOVERNMENT RIGHTS

This invention was made with United States government support under Grants CA-32447 and CA-16885 from the National Cancer Institute. The United States Government has certain rights in this invention.

## Claims

1. A pharmaceutical composition for treating a patient infected with LAV/HTLV III virus comprising in a therapeutically effective amount an agent inhibiting the production and/or the activity of mononuclear cell derived cytotoxic factors, without permanently interfering with other immune cell functions.

2. The composition according to claim 1, wherein the mononuclear cell derived cytotoxic factors are comprised by the group consisting of lymphotoxin and tumor necrosis factor.

3. The composition according to claim 1, wherein the mononuclear cell derived cytotoxic factors are comprised by the group consisting of leukoregulin and natural killer cytotoxic factor.

4. The composition according to claim 1, wherein said mononuclear cells are comprised by the group consisting of thymus derived lymphocytes and monocytes.

5. The composition according to claim 1, wherein the agent for inhibiting the activity of cytotoxic factors comprises monoclonal or polyclonal antibodies.

6. The composition according to claim 1, wherein the agent for the inhibition of the activity of the cytotoxic factors comprises an agent for blocking the cellular receptors of the cytotoxic factors.

7. The composition according to claim 6, wherein said blocking agent is an antagonist to the receptors.

8. The composition according to claim 7, wherein said antagonist is selected from the group consisting of a monoclonal or polyclonal antibody to the receptor, monosaccharides, oligosaccharides, polysaccharides, derivatives of monosaccharides, derivatives of oligosaccharides, derivatives of polysaccharides and nonfunctional cytotoxin molecules.

9. A pharmaceutical composition comprising (a) interleukin 2 and (b) cyclosporin or steroids in a pharmaceutically acceptable carrier for use in the treatment of acquired immunodeficiency syndrome.

10. A composition according to claim 9, wherein the steroid is imuran.

11. A composition according to claim 9, wherein the steroid is prednisone.

12. A composition comprising lymphotoxin lacking the N-terminal end and containing 15 to 20 C-terminal amino acids in a pharmaceutically acceptable carrier for use in the treatment of acquired immunodefficiency syndrome.

13. The use of a composition comprising (a) interleukin 2 and (b) cyclosporin or steroids to prepare a pharmaceutical suitable for treating a patient infected with LAV/HTLV III virus.

14. A use according to claim 13, wherein the steroid is imuran.

15. A use according to claim 13, wherein the steroid is prednisone.

16. The use of an effective amount of lymphotoxin lacking the N-terminal end and containing 15 to 20 C-terminal amino acids to prepare a pharmaceutical suitable for treating a patient infected with LAV/HTLV III virus.

Claims for the following contracting states: AT, ES, GR

1. A method for producing a pharmaceutical composition for treating a patient infected with LAV/HTLV III virus characterized by mixing a compound which inhibits the production and/or the activity of mononuclear cell derived cytotoxic factors, without permanently interfering with other immune cell functions, in a therapeutically effective amount with a suitable carrier or diluent.

2. The method according to claim 1, wherein the mononuclear cell derived cytotoxic factors are comprised by the group consisting of lymphotoxin and tumor necrosis factor.

3. The method according to claim 1, wherein the mononuclear cell derived cytotoxic factors are comprised by the group consisting of leukoregulin and natural killer cytotoxic factor.

4. The method according to claim 1, wherein said mononuclear cells are comprised by the group consisting of thymus derived lymphocytes and monocytes.

5. The method according to claim 1, wherein the inhitition of the activity of cytotoxic factors is achieved by an effective amount of monoclonal or polyclonal antibodies to said cytotoxic factors.

6. The method according to claim 1, wherein the agent for the inhibition of the activity of the cytotoxic factors comprises an agent for blocking the cellular receptors of the cytotoxic factors.

7. The method according to claim 6, wherein said blocking is effected by an effective amount of antagonist to the receptors.

8. The method according to claim 7, wherein said antagonist is selected from the group consisting of a monoclonal or polyclonal antibody to the receptor, monosaccharides, oligosaccharides, polysaccharides, derivatives of monosaccharides, derivatives of oligosaccharides, derivatives of polysaccharides and nonfunctional cytotoxin molecules.

9. A method for the preparation of a pharmaceutical composition, characterized in that (a) interleukin 2 and (b) cyclosporin or steroids are mixed in a pharmaceutically acceptable carrier for use in the treatment of acquired immunodeficiency syndrome.

10. The method according to claim 9, wherein the steroid is imuran.

11. The method according to claim 9, wherein the steroid is prednisone.

12. A method for preparing a composition for use in the treatment of acquired immunodeficiency syndrome, characterized in that lymphotoxin lacking the N-terminal end and containing 15 to 20 C-terminal amino acids is mixed with a pharmaceutically acceptable carrier.

NORMAL LYMPHOCYTES INOCULATED WITH H9 HTLV III

FIG.1

FIG.2

FIG.3